(19) Europäisches Patentamt
European Patent Office
Office européen des brevets

(11) **EP 2 134 465 B1**

(12) **EUROPÄISCHE PATENTSCHRIFT**

(45) Veröffentlichungstag und Bekanntmachung des Hinweises auf die Patenterteilung:
**13.06.2018   Patentblatt 2018/24**

(21) Anmeldenummer: **08717204.5**

(22) Anmeldetag: **28.02.2008**

(51) Int Cl.:
*B01J 23/00* *(2006.01)*          *B01J 23/28* *(2006.01)*
*B01J 23/888* *(2006.01)*        *B01J 37/02* *(2006.01)*
*C07C 51/25* *(2006.01)*

(86) Internationale Anmeldenummer:
**PCT/EP2008/052402**

(87) Internationale Veröffentlichungsnummer:
**WO 2008/104577 (04.09.2008 Gazette 2008/36)**

(54) **VERFAHREN ZUR HERSTELLUNG EINES KATALYSATORS BESTEHEND AUS EINEM TRÄGERKÖRPER UND EINER AUF DER OBERFLÄCHE DES TRÄGERKÖRPERS AUFGEBRACHTEN KATALYTISCH AKTIVEN MASSE**

METHOD FOR PRODUCING A CATALYST CONSISTING OF A CARRIER BODY AND A CATALYTICALLY ACTIVE MASS APPLIED TO THE SURFACE OF THE CARRIER BODY

PROCÉDÉ DE FABRICATION D'UN CATALYSEUR COMPOSÉ D'UN CORPS SUPPORT ET D'UNE MASSE À ACTIVITÉ CATALYTIQUE APPLIQUÉE SUR LA SURFACE DU CORPS SUPPORT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **01.03.2007   DE 102007010422**
**01.03.2007   US 892419 P**

(43) Veröffentlichungstag der Anmeldung:
**23.12.2009   Patentblatt 2009/52**

(73) Patentinhaber: **BASF SE**
**67056 Ludwigshafen am Rhein (DE)**

(72) Erfinder:
• **CREMER, Ulrich**
**68161 Mannheim (DE)**
• **RAICHLE, Andreas**
**67063 Ludwigshafen (DE)**
• **ROSOWSKI, Frank**
**68239 Mannheim (DE)**
• **HAMMON, Ulrich**
**68163 Mannheim (DE)**
• **MÜLLER-ENGEL, Klaus Joachim**
**76297 Stutensee (DE)**

(74) Vertreter: **BASF IP Association**
**BASF SE**
**G-FLP-C006**
**67056 Ludwigshafen (DE)**

(56) Entgegenhaltungen:
WO-A-02/24327          DE-A1- 10 350 822
DE-A1-102004 025 445

**Beschreibung**

Die vorliegende Erfindung betrifft ein Verfahren zur Herstellung eines Katalysators (eines Schalenkatalysators) bestehend aus einem Trägerkörper und einer auf der Oberfläche des Trägerkörpers aufgebrachten katalytisch aktiven Masse, bei dem man die aktive Masse mit Hilfe eines Bindemittels an die Oberfläche des Trägerkörpers anheftet.

Verfahren zur Herstellung von Schalenkatalysatoren der vorbeschriebenen Art und Weise sind bekannt (vgl. z. B. WO 95/11081, WO 2004/108267, Wo 2004/108284, US-A 2006/0205978, EP-A 714700 und DE-A 102005010645).

Die aktive Masse ist dabei in einer großen Anzahl von Fällen ein die Elemente Mo und V enthaltendes Multielementoxid. Die Bezeichnung Multielementoxid bringt dabei zum Ausdruck, dass die aktive Masse neben Mo, V und O (Sauerstoff) noch wenigstens ein weiteres chemisches Element enthält.

Der molare Anteil des Elementes Mo an der Gesamtmenge aller von Sauerstoff verschiedenen Elemente der katalytisch aktiven Multielementoxidmasse beträgt dabei in der Regel 5 bis 95 mol-%, häufig 10 bis 90 mol-% und vielfach 15 bis 85 mol-% bzw. 20 bis 80 mol-%. Das molare Verhältnis von in der katalytisch aktiven Multielementoxidmasse enthaltenem Mo zu in der katalytisch aktiven Multielementoxidmasse enthaltenem V, Mo/V, beträgt üblicherweise 15:1 bis 1:1, häufig 12:1 bis 2:1.

Katalysatoren der vorbeschriebenen Art eignen sich insbesondere für die Katalyse der heterogen katalysierten partiellen Gasphasenoxidation von Acrolein zu Acrylsäure.

Aus der DE-A 10350822 und aus der DE-A 102004025445 ist bekannt, dass ein solches Verfahren der heterogen katalysierten Gasphasenpartialoxidation von Acrolein zu Acrylsäure an ein und demselben Katalysatorfestbett im wesentlichen kontinuierlich über längere Zeiträume betrieben werden kann.

Allerdings verliert das Katalysatorfestbett dabei im Verlauf der Betriebszeit an Qualität. Im besonderen verschlechtert sich seine Aktivität.

Um das Katalysatorfestbett, dessen Fertigung und Austausch vergleichsweise aufwendig und kostspielig sind, trotzdem möglichst lange in einem damit beschickten Reaktor betreiben zu können, wird im Stand der Technik auf unterschiedlichste Art und Weise versucht, dem Alterungsprozess des Katalysatorfestbetts entgegenzuwirken.

Die EP-A 990636 (z. B. Seite 8, Zeilen 13 bis 15) und die EP-A 1106598 (z. B. Seite 13, Zeilen 43 bis 45) schlagen vor, die Minderung der Aktivität des Katalysatorfestbetts dadurch weitgehend zu kompensieren, dass im Verlauf der Betriebszeit unter ansonsten weitgehend gleichbleibenden Betriebsbedingung, die Temperatur das Katalysatorfestbetts nach und nach erhöht wird, um den Acroleinumsatz bei einmaligem Durchgang des Reaktionsgasgemischs durch das Katalysatorfestbett im wesentlichen beizubehalten.

Nachteilig an der in der EP-A 990636 sowie in der EP-A 1106598 empfohlenen Verfahrensweise ist, das sich mit zunehmender Erhöhung der Temperatur des Katalysatorfestbetts sein Alterungsprozess zunehmend beschleunigt (bestimmte Bewegungsprozesse innerhalb der Katalysatoren, die zur Alterung beitragen, laufen z. B. zunehmend schneller ab). Bei Erreichen eines Höchstwerts der Temperatur des Katalysatorfestbetts muss das Katalysatorfestbett schließlich vollständig ausgetauscht werden.

Nachteilig an einem solchen Komplettaustausch ist jedoch, dass er vergleichsweise aufwendig ist. Das Verfahren der Acrylsäureherstellung muss für längere Zeit unterbrochen werden und die Kosten der Katalysatorherstellung sind ebenfalls erheblich.

Erwünscht sind daher Verfahrenweisen, die dabei behilflich sind, die Standzeit des Katalysatorfestbetts im Reaktor weitestgehend in die Länge zu ziehen.

Die DE-A 10232748 empfiehlt diesbezüglich, anstatt das Katalysatorfestbett vollständig auszutauschen, nur eine Teilmenge desselben durch eine frische Katalysatorbeschickung zu ersetzen. Nachteilig an dieser Verfahrensweise ist, dass auch ein Katalysatorbettteilwechsel bereits vergleichsweise aufwendig ist und eine Unterbrechung der Partialoxidation von Acrolein zu Acrylsäure erfordert.

Die DE-A 102004025445 schlägt als ein Verfahren zum Langzeitbetrieb der heterogen katalysierten partiellen Gasphasenoxidation von Acrolein zu Acrylsäure vor, der Deaktivierung des Katalysatorfestbetts dadurch entgegenzuwirken, dass der Arbeitsdruck in der Gasphase mit zunehmender Betriebsdauer des Katalysatorfestbetts zunehmend erhöht wird. Nachteilig an dieser Verfahrensweise ist, dass mit zunehmendem Arbeitsdruck bei der heterogen katalysierten partiellen Gasphasenoxidation erhöhte Kompressionsleistungen erforderlich sind.

Die EP-A 614872 empfiehlt, die Standzeit des Katalysatorfestbetts dadurch zu verlängern, dass man nach mehrjähriger Betriebsdauer des Katalysatorfestbetts, mit der Erhöhungen der Temperatur desselben von 15 °C bis 30 °C und mehr einhergehen, das Verfahren der Partialoxidation unterbricht, und bei erhöhter Temperatur des Katalysatorfestbetts durch selbiges ein Regeniergasgemisch aus Sauerstoff, Wasserdampf und Inertgas führt und anschließend die Partialoxidation fortsetzt (in diesem Zusammenhang sollen in dieser Schrift als Inertgase in einem Gasgemisch, das unter bestimmten Bedingungen durch ein Katalysatorfestbett geführt wird, ganz generell solche Gase verstanden werden, die bei der Durchführung des Gasgemischs durch das Katalysatorfestbett zu wenigstens 95 mol-%, bevorzugt zu wenigstens 98 mol-%, ganz besonders bevorzugt zu wenigstens 99 mol-% oder 99,5 mol-% unverändert erhalten bleiben).

Nachteilig an der Verfahrensweise der EP-A 614872 ist jedoch, dass bis zum Zeitpunkt der Unterbrechung die Alterung des Katalysatorfestbetts ungebremst fortschreitet und gefördert wird.

Die DE-A 10350822 versucht dem Nachteil der EP-A 614872 dadurch abzuhelfen, dass über die Betriebszeit zum Ausgleich der Deaktivierung des Katalysatorfestbetts die Temperatur des Katalysatorfestbetts zwar erhöht wird, bevor diese Temperaturerhöhung jedoch 8 °C beträgt wird die Partialoxidation unterbrochen und in regenerierender weise ein Sauerstoff enthaltendes Gas durch das Katalysatorfestbett geführt. Nachteilig an der Verfahrensweise der DE-A 10350822 ist jedoch, dass jede Regenerierung eine Unterbrechung des eigentlichen Partialoxidationsverfahrens erforderlich macht.

WO-A-02/24327 beschreibt ein Verfahren zur Herstellung von Multimetalloxidmassen, die als aktive Masse von Katalysatoren insbesondere zur katalytischen Oxidation organischer Verbindungen in der Gasphase Verwendung finden, die dadurch erhältlichen Multimetalloxidmassen und ein Verfahren zur Herstellung von Acrylsäure unter Verwendung von Katalysatoren, die diese Multimetalloxidmassen als aktive Masse enthalten.

Nachteilig an allen vorstehend gewürdigten Verfahren des Standes der Technik zur Verlängerung der Standzeit eines zur Durchführung einer heterogen katalysierten Partialoxidation von Acrolein zu Acrylsäure geeigneten Katalysatorfestbetts ist zusätzlich, dass sie allesamt nicht vorab präventiv dem Eintreten einer Deaktivierung des Katalysatorfestbetts entgegenzuwirken versuchen, sonder erst dann einsetzen, wenn eine solche Deaktivierung des Katalysatorfestbetts bereits eingetreten ist, um der negativen Folgewirkung einer solchen Deaktivierung entgegenzuwirken.

Die Aufgabe der vorliegenden Erfindung bestand daher darin, ein Verfahren zur Verfügung zu stellen, das dazu geeignet ist, einer Deaktivierung von Schalenkatalysatoren, deren Aktivmasse ein auf einen Trägerkörper aufgebrachtes Mo und V enthaltendes feinteiliges Multielementoxid ist, im Verlauf einer heterogen katalysierten partiellen Gasphasenoxidation von Acrolein zu Acrylsäure präventiv entgegenzuwirken (d. h., das Einsetzen der Deaktivierung hinauszuzögern).

Als Lösung der Aufgabe wurde ein Verfahren zur Herstellung eines Katalysators bestehend aus einem Trägerkörper und einer auf der Oberfläche des Trägerkörpers aufgebrachten katalytisch aktiven Masse, bei dem man die aktive Masse mit Hilfe eines Bindemittels an die Oberfläche des Trägerkörpers anhaftet, gefunden, das dadurch gekennzeichnet ist, dass die aktive Masse ein feinteiliges Gemisch aus

- wenigstens einem die Elemente Mo und V enthaltenden feinteiligen Multielementoxid
  und

- wenigstens einer feinteiligen Substanz S ausgewählt aus der Gruppe bestehend aus Oxiden des Molybdäns und aus Verbindungen des Molybdäns, aus denen sich unter Einwirkung von erhöhter Temperatur und molekularem Sauerstoff ein Oxid des Molybdäns bildet, ist,

wobei unter einem Oxid des Molybdäns eine Substanz verstanden wird, die zu $\geq$ 98 Gew.-% aus Mo und O besteht. Vorteilhaft an der erfindungsgemäßen Verfahrensweise ist, dass das Mo und V enthaltende feinteilige Multielementoxid vorab der Beschichtung des Trägerkörpers mit der katalytisch aktiven Masse separat hergestellt wird. Dies hat zur Folge, dass seine katalytische Wirksamkeit bezüglich der partiellen Oxidation des Acroleins zu Acrylsäure durch das Zumischen der feinteiligen Substanz S im wesentlichen nicht beeinträchtigt wird.

Handelt es sich bei der feinteiligen Substanz S nicht bereits um ein Oxid des Molybdäns, so kann erfindungsgemäß alternativ eine Verbindung des Molybdäns verwendet werden, aus der sich unter der Einwirkung von erhöhter Temperatur und molekularem Sauerstoff ein Oxid des Molybdäns bildet. Die Einwirkung der erhöhten Temperatur und des molekularen Sauerstoff kann z. B. im Anschluss an das Aufbringen des feinteiligen aktiven Gemischs auf die Oberfläche des Trägerkörpers erfolgen. Anwendungstechnisch zweckmäßig wird die dabei angewandte Temperatur unterhalb der höchsten zur Herstellung des Mo und V enthaltenden Multielementoxids angewandten Temperatur liegend gewählt. Zur Bereitstellung des molekularen Sauerstoff kann die thermische Behandlung z. B. unter molekularem Sauerstoff oder unter einem Gemisch aus molekularem Sauerstoff und Inertgas (z. B. Luft) erfolgen.

Als Beispiele für erfindungsgemäß geeignete feinteilige Substanzen S, die von einem Oxid des Molybdäns verschieden sind, seien das Ammoniummolybdat [(NH$_4$)$_2$MoO$_4$] sowie die Ammoniumpolymolybdate wie das Ammoniumheptamolybdattetrahydrat [(NH$_4$)$_6$Mo$_7$O$_{24}$ • 4 H$_2$O] genannt. Ein alternatives Beispiel ist Molybdänoxidhydrat (MoO$_3$ • xH$_2$O). Aber auch Molybdänhydroxide kommen als solche Substanzen S in Betracht.

Die Einwirkung der erhöhten Temperatur und des molekularen Sauerstoff auf die von einem Molybdänoxid verschiedene Substanz S kann aber auch erst durch die heterogen katalysierte Partialoxidation von Acrolein zu Acrylsäure selbst erfolgen. In diesem Fall erfolgt die Molybdänoxidbildung erst während der Durchführung der heterogen katalysierten partiellen Oxidation von Acrolein zu Acrylsäure unter Verwendung von erfindungsgemäß hergestellten Schalenkatalysatoren. Erfindungsgemäß bevorzugt wird man als feinteilige Substanz S jedoch ein Oxid (z. B. MoO$_2$) des Molybdäns einsetzen (darunter wird in dieser Schrift eine Substanz verstanden, die zu $\geq$ 98 Gew.-%, vorzugsweise zu $\geq$ 99 Gew.-% und besonders bevorzugt zu $\geq$ 99,9 Gew.-% und mehr nur aus Mo und O besteht). Besonders bevorzugt wird man beim erfindungsgemäßen Verfahren als feinteilige Substanz S Molybdäntrioxid (MoO$_3$) verwenden.

Grundsätzlich kommen als feinteilige Substanz S aber auch Molybdänoxide wie $Mo_{18}O_{52}$, $Mo_8O_{23}$ und $Mo_4O_{11}$ in Betracht (vgl. z. B. "Synthese und strukturelle Untersuchungen von Molybdän-, Vanadium- und Wolframoxiden als Referenzverbindungen für die heterogene Katalyse", Dissertation von Dr. Andreas Blume, Fakultät II Mathematik- und Naturwissenschaften der Technischen Universität Berlin, 2004, oder Surface Science 292 (1993) 261-6, oder J. Solid State Chem. 124 (1996) 104).

Anwendungstechnisch zweckmäßig beträgt die spezifische Oberfläche $O_M$ eines im erfindungsgemäßen Verfahren als feinteilige Substanz S vorteilhaft eingesetzten Molybdänoxids erfindungsgemäß bevorzugt $\leq 10$ m$^2$/g, besonders bevorzugt $\leq 5$ m$^2$/g und ganz besonders bevorzugt $\leq 2$ m$^2$/g. In der Regel wird die spezifische Oberfläche $O_M$ jedoch $\geq 0,01$ m$^2$/g, häufig $\geq 0,05$ m$^2$/g und vielfach $\geq 0,1$ m$^2$/g betragen.
Unter der spezifischen Oberfläche wird dabei die BET-Oberfläche verstanden (bestimmt durch Gasadsorption ($N_2$) nach Brunauer-Emmet-Teller (BET)).
Die vorgenannten Aussagen bezüglich $O_M$ treffen insbesondere dann zu, wenn das feinteilige Molybdänoxid $MoO_3$ ist. Die Vorteilhaftigkeit eines geringen Wertes für $O_M$ liegt darin begründet, dass ein Molybdänoxid mit einem niedrigen Wert für $O_M$ sich im Rahmen einer heterogen katalysierten partiellen Gasphasenoxidation von Acrolein zu Acrylsäure weitgehend inert verhält. D. h., erfindungsgemäß besonders vorteilhaft wird man als feinteilige Substanz S solches Molybdänoxid (insbesondere $MoO_3$) verwenden, das so beschaffen ist, dass wenn man den Schalenkatalysator (gleicher Trägerkörper, gleiche Schalendicke, gleiche Partikelgröße der auf den Träger aufgebrachten feinteiligen Masse) unter alleiniger Verwendung des Molybdänoxids (insbesondere $MoO_3$) als "aktive Masse" herstellt, unter den Bedingungen, unter denen bei Verwendung eines Schalenkatalysators der unter alleiniger Verwendung des das die Elemente Mo und V enthaltenden feinteiligen Multielementoxids als aktive Masse hergestellt wurde, ein Acroleinumsatz von 95 bis 100 mol-% (bezogen auf einmaligen Durchgang des Reaktionsgasgemischs durch das mit dem jeweiligen Schalenkatalysator in ansonsten identischer Weise beschickte Katalysatorfestbett) erzielt wird, lediglich ein Acroleinumsatz von $\leq 10$ mol-%, bevorzugt von $\leq 5$ mol-% und ganz besonders von $\leq 2$ mol-% bzw. von $\leq 1$ mol-% erzielt wird.

Die Körnung (Partikeldurchmesser, bzw. Partikeldurchmesserverteilung) der feinteiligen Substanz S ist erfindungsgemäß vorteilhaft mit jener des die Elemente Mo und V enthaltenden feinteiligen Multielementoxids identisch (dies ermöglicht eine besonders homogene Vermischung mit dem feinteiligen Multielementoxid). Dies gilt insbesondere dann, wenn die feinteilige Substanz S ein Molybdänoxid (insbesondere $MoO_3$) ist.

Selbstredend kann die Körnung der feinteiligen Substanz S bei erfindungsgemäßen Verfahren aber auch von jener des die Elemente Mo und V enthaltenden feinteiligen Multielementoxids verschieden sein.

Partikeldurchmesserverteilungen sowie aus diesen entnommene Partikeldurchmesser $d_x$ (z. B. $d_{10}$, oder $d_{50}$, oder $d_{90}$) beziehen sich auf Bestimmungen nach ISO 13320 mit dem Laserbeugungsspektrometer Malvern Mastersizer S (Malvern Instruments, Worcestshire WR 14 1AT, United Kingdom). Die als Messergebnis angegebenen Partikeldurchmesser $d_x$ sind dabei so definiert, dass X% des Gesamtpartikelvolumens aus Partikeln mit diesem oder einem kleinerem Durchmesser bestehen.

Zur Bestimmung von Partikeldurchmesserverteilungen wird das jeweilige feinteilige Pulver anwendungstechnisch zweckmäßig über eine Dispergierrinne in den Trockendispergierer Sympatec RODOS (Sympatec GmbH, System-Partikel-Technik, Am Pulverhaus 1, D-38678 Clausthal-Zellerfeld) geführt und dort mit Druckluft trocken dispergiert und im Freistrahl in die Messzelle geblasen. In dieser erfolgt dann die eigentliche Laserbeugungsbestimmung.

Ganz generell sollte die Körnung der erfindungsgemäß mitzuverwendenden feinteiligen Substanz S (insbesondere dann, wenn es sich um ein Molybdänoxid (z. B. um $MoO_3$) handelt) beim erfindungsgemäßen Verfahren so beschaffen sein, dass die Längstausdehnung $d_L$ (längste direkte Verbindungslinie zweier auf der Partikeloberfläche befindlicher Punkte) von $\geq 50$ %, vorzugsweise von $\geq 75$ % des Gesamtvolumens aller Partikel $\leq 800$ $\mu$m, vorteilhaft $\leq 600$ $\mu$m, besonders vorteilhaft $\leq 400$ $\mu$m oder $\leq 300$ $\mu$m und ganz besonders vorteilhaft $\leq 200$ $\mu$m oder $\leq 100$ $\mu$m beträgt.

In der Regel wird die Körnung der erfindungsgemäß mitzuverwendenden feinteiligen Substanz S (insbesondere dann, wenn es sich um eine Molybdänoxid (z. B. um $MoO_3$) handelt) bei erfindungsgemäßen Verfahren so beschaffen sein, dass $d_L$ von $\geq 50$ %, vorzugsweise von $\geq 75$ % des Gesamtvolumens aller Partikel $\geq 0,1$ $\mu$m, häufig $\geq 0,5$ $\mu$m und vielfach $\geq 1$ $\mu$m betragen.

D. h., für das erfindungsgemäße Verfahren kommen insbesondere solche feinteiligen Substanzen S (insbesondere Molybdänoxide (z. B. $MoO_3$)) in Betracht, für die 0,1 $\mu$m $\leq d_{50} \leq 800$ $\mu$m, mit Vorteil 0,5 $\mu$m $\leq d_{50} \leq 600$ $\mu$m, vorzugsweise 0,75 $\mu$m $\leq d_{50} \leq 400$ $\mu$m (bzw. $\leq 300$ $\mu$m), besonders bevorzugt 1 $\mu$m $\leq d_{50} \leq 200$ $\mu$m (bzw. $\leq 100$ $\mu$m) beträgt.

Grundsätzlich wird beim erfindungsgemäßen Verfahren die Körnung der feinteiligen Substanz S (insbesondere im Fall eines Molybdänoxids (z. B. $MoO_3$) an die angestrebte Dicke $D_A$ der Aktivmassenschale auf der Oberfläche des Trägerkörpers angepasst.

D. h., in der Regel wird $d_{50} \leq D_A$, vorzugsweise $\leq 0,75 \cdot D_A$, besonders bevorzugt $\leq 0,5 \cdot D_A$ und ganz besonders bevorzugt $\leq 0,3 \cdot D_A$ betragen.

Normalerweise wird $d_{50}$ jedoch $\geq 0,001 \cdot D_A$, bzw. $\geq 0,01 \cdot D_A$, häufig $\geq 0,05 \cdot D_A$ und vielfach $\geq 0,1 \cdot D_A$ betragen.

Die Gesamteinsatzmenge an feinteiliger Substanz S wird, bezogen auf die Gesamteinsatzmenge an Mo und V enthaltendem feinteiligem Multielementoxid, beim erfindungsgemäßen Verfahren anwendungstechnisch zweckmäßig > 0 und ≤ 50 Gew.-% betragen. Erfindungsgemäß vorteilhaft wird die vorgenannte, in gleicher Weise bezogene, Einsatzmenge ≥ 0,1, meist ≥ 0,5 und häufig ≥ 1 Gew.-% betragen.

Vielfach wird die vorgenannte in gleicher Weise bezogene Einsatzmenge ≤ 40 Gew.-% oder ≤ 30 Gew.-% betragen. Erfindungsgemäß bevorzugt wird die vorgenannte, in gleicher Weise bezogene, Einsatzmenge bei ≥ 5 und ≤ 20 Gew.-% (bzw. ≤ 15 Gew.-%) liegen. Die vorstehenden Mengenangaben treffen insbesondere dann zu, wenn die feinteilige Substanz S ein Molybdänoxid (z. B. $MoO_3$) ist.

Grundsätzlich kann ein für das erfindungsgemäße Verfahren als feinteilige Subsatz S geeignetes Molybdänoxid (z.B. $MoO_3$) aus einer anderen Mo enthaltenden Substanz gezielt erzeugt werden.

Zu diesem Zweck kann z. B. von Ammoniumheptamolybdattetrahydrat [$(NH_4)_6Mo_7O_{24} \cdot 4\ H_2O$] ausgegangen werden. Durch z. B. 3-stündiges thermisches Behandeln bei 350 °C im ebenfalls eine Temperatur von 350 °C aufweisenden Luftstrom wird dieses in $MoO_3$ gewandelt. Die Körnung des $MoO_3$ kann durch entsprechendes Mahlen und Sieben in beliebiger Weise bedarfsgerecht eingestellt werden. In entsprechender Weise kann auch die spezifische Oberfläche des $MoO_3$ nach Wunsch eingestellt werden. Mit zunehmender Dauer der thermischen Behandlung und/oder Erhöhung der Temperatur der thermischen Behandlung (nach erfolgter $MoO_3$ Ausbildung unter Inertgas oder unter molekularen Sauerstoff enthaltender Gasatmosphäre (z. B. Luft)) nimmt die spezifische Oberfläche ab.

Nach erfolgter Ausbildung des $MoO_3$ bei 350 °C ist in der Regel ein 4- bis 8-stündiges thermisches Behandeln bei 550 bis 650 °C im eine entsprechende Temperatur aufweisenden Luftstrom ausreichend, um die spezifische Oberfläche $O_M$ des $MoO_3$ auf einen Wert von ≤ 2 m²/g zu drücken.

Selbstredend können für das erfindungsgemäße Verfahren als feinteilige Substanz S geeignete Molybdänoxide aber auch im Handel erworben werden.

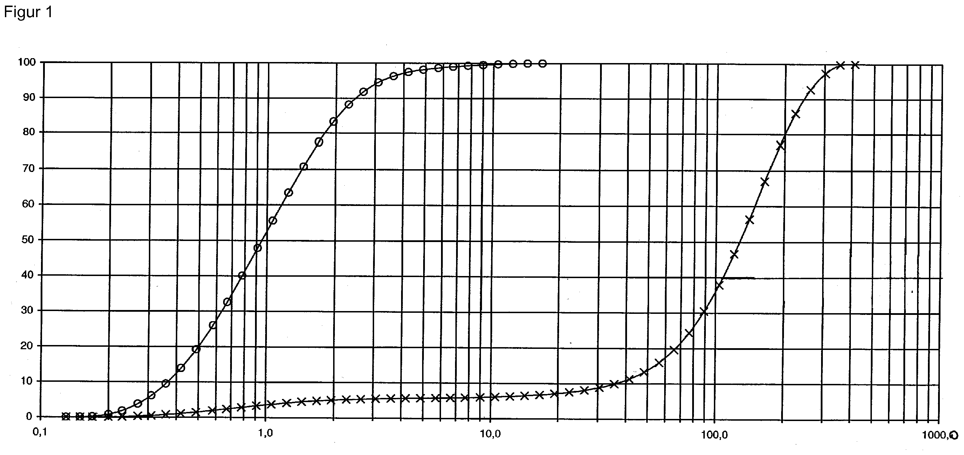

Beispielsweise eignet sich für das erfindungsgemäße Verfahren $MoO_3$ der Climax Molybdenum Marketing Corporation (Phoenix, USA), das einen Mo-Gehalt von 66,60 Gew.-% und eine spezifische Oberfläche $O_M$ von 3,7 m²/g aufweist (Handelsname: "pure Moly Oxide Crystalline POC"). Figur 1 zeigt die für dieses $MoO_3$ nach ISO 13320 bestimmte Partikeldurchmesserverteilung (Laser, Malvern). Dabei zeigt die Abszisse die Durchmesser [μm] im logarithmischen Maßstab. Die Ordinate zeigt den Volumenanteil des $MoO_3$, der den jeweiligen Durchmesser oder einen kleineren Durchmesser aufweist. Das käuflich zu erwerbende Produkt weist die in Figur 1 gezeigte Partikeldurchmesserverteilung X auf. Bei diesen Partikeln handelt es sich jedoch um Agglomerate aus Primärpartikeln. Durch Einwirkung von z. B. Ultraschall kann der Zerfall der Agglomerate in die Primärpartikel bewirkt werden. Diese haben die in Figur 1 gezeigte Partikeldurchmesserverteilung O. Für das erfindungsgemäße Verfahren kommen alle Partikeldurchmesserverteilungen in Betracht, die durch Abmischen der in Figur 1 gezeigten Partikeldurchmesserverteilungen X und O (in beliebigen Mengenverhältnissen (z. B. 1000 : 1 bis 1 : 1000, oder 100 : 1 bis 1 : 100, oder 10 : 1 bis 1 : 10, oder 5 : 1 bis 1 : 5, oder 2 : 1 bis 1 : 2)) erzeugt werden können. In der Praxis können diese Partikeldurchmesserverteilungen z. B. dadurch erzeugt werden, dass man Primärpartikel und Agglomerat im entsprechenden (Gewichts)Mengenverhältnis miteinander vermischt.

In der Regel weist das vorgenannte $MoO_3$ zusätzlich die nachfolgende Fremdbestandteilsspezifikation auf:

| | |
|---|---|
| Na | ≤ 8 Gew.-ppm, |
| K | ≤ 29 Gew.-ppm, |
| Fe | ≤ 4 Gew.-ppm, |
| Pb | ≤ 1 Gew.-ppm, |
| Al | ≤ 4 Gew.-ppm, |
| Cr | ≤ 2 Gew.-ppm, |
| Ca | ≤ 2 Gew.-ppm, |
| Cu | ≤ 2 Gew.-ppm, |
| Mg | ≤ 5 Gew.-ppm, |
| Ni | ≤ 2 Gew.-ppm, |
| Si | ≤ 5 Gew.-ppm, |
| Sn | ≤ 1 Gew.-ppm, und |
| Ti | ≤ 2 Gew.-ppm. |

Selbstverständlich kann aber auch $MoO_3$ der Climax Molybdenum Marketing Corporation vom Handelstyp "POS" erfindungsgemäß eingesetzt werden.

Alternativ kann als käuflich erwerbliches $MoO_3$ auch $MoO_3$ der Fa. H. C. Starck, D-38615 Goslar für das erfindungsgemäße Verfahren verwendet werden (Handelsname: "Molybdenum Trioxide I").

Dieses besitzt eine spezifische Oberfläche $O_M$ von 1 m²/g. Der Mo-Gehalt dieses $MoO_3$ liegt bei 66,6 Gew.-%.

Im übrigen weist dieses erfindungsgemäß geeignete $MoO_3$ der Fa. H. C. Starck die nachfolgende Fremdkom-

ponentenspezifikation auf:

| | |
|---|---|
| $NH_4$ | $\leq$ 0,01 Gew.-%, |
| Al | $\leq$ 10 Gew.-ppm, |
| Ca | $\leq$ 5 Gew.-ppm, |
| Co | $\leq$ 10 Gew.-ppm, |
| Cr | $\leq$ 5 Gew.-ppm, |
| Cu | $\leq$ 5 Gew.-ppm, |
| Fe | $\leq$ 10 Gew.-ppm, |
| K | $\leq$ 80 Gew.-ppm, |
| Mg | $\leq$ 5 Gew.-ppm |
| Mn | $\leq$ 10 Gew.-ppm, |
| Na | $\leq$ 20 Gew.-ppm, |
| Ni | $\leq$ 5 Gew.-ppm, |
| P | $\leq$ 10 Gew.-ppm, |
| Pb | $\leq$ 10 Gew.-ppm, |
| Si | $\leq$ 10 Gew.-ppm, |
| Sn | $\leq$ 10 Gew.-ppm, |
| Ti | $\leq$ 5 Gew.-ppm, |
| V | $\leq$ 10 Gew.-ppm, |
| Zn | $\leq$ 10 Gew.-ppm, und |
| Zr | $\leq$ 10 Gew.-ppm. |

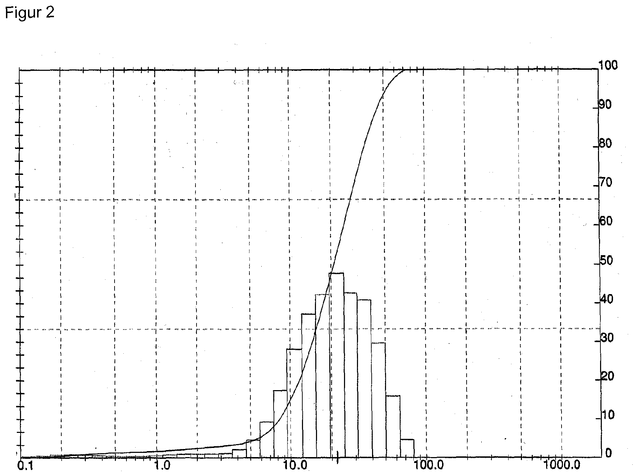

Die zugehörige Partikeldurchmesserverteilung zeigt Figur 2. Dabei zeigt die Abszisse die Durchmesser [$\mu$m] im logarithmischen Maßstab. Die Ordinate zeigt den Volumenanteil des $MoO_3$, der den jeweiligen Durchmesser oder einen kleineren Durchmesser aufweist.

Bei den $MoO_3$-Partikeln des $MoO_3$ der Fa. H. C. Starck handelt es sich ebenfalls um Agglomerate aus Primärpartikeln. Im Unterschied zu den $MoO_3$-Partikeln des $MoO_3$ der Fa. Climax ist der Zusammenhalt der Primärpartikel jedoch wesentlich ausgeprägter, weshalb durch Einwirkung von z. B. Ultraschall kein Zerfall in die Primärpartikel bewirkt werden konnte.

Selbstverständlich kann aber auch Molybdenum Trioxide der "II"-Typen der Fa. H. C. Starck erfindungsgemäß verwendet werden.

Im übrigen kann für das erfindungsgemäße Verfahren aber auch $MoO_3$ der folgenden Hersteller eingesetzt werden:

- Fa. Metal-Tech.-Ltd. (Israel), Reinheit > 98 Gew.-%, $O_M$ = 1,1 $m^2$/g;
- Gulf Chemical (Texas, USA), 65,76 Gew.-% Mo, $O_M$ = 1,2 $m^2$/g;
- Nanjing Chemical Industries (China), 66,6 Gew.-% Mo, $O_M$ = 0,8 $m^2$/g;
- Kankal Exports (Indien), Reinheit $\geq$ 99 Gew.-%, $O_M$ = 1,7 $m^2$/g;
- Taiyo Koko Co., Ltd. (Japan), Reinheit $\geq$ 99,7 Gew.-%, $O_M$ = 1,6 $m^2$/g;
- Anhui Chizhou Huangshanling Lead and Zinc Mine (China), Reinheit $\geq$ 99,7 Gew.-%, 66,5 Gew.-% Mo, $O_M$ = 0,3 $m^2$/g;
- CCI Moly B.V. (Niederlande), Reinheit > 99,5 Gew.-%, > 66 Gew.-% Mo, $O_M$ = 2,5 $m^2$/g.

Als feinteiliges, die Elemente Mo und V enthaltendes, Multielementoxid kommen alle im Stand der Technik bekannten Multielementoxidmassen in Betracht, die die Partialoxidation von Acrolein zu Acrylsäure zu katalysieren vermögen.

Dies sind insbesondere die Mo und V enthaltenden Multielementoxidmassen der DE-A 102005010645, der WO 95/11081, der DE-A 10350822, der US-A 2006/0205978, der EP-A 714700, der DE-A 102004025445, der WO 2004/108267, der WO 2004/108284 sowie aller in den vorgenannten Schriften als Stand der Technik zitierten Schriften. Besonders bevorzugte Mo und V enthaltende Multielementoxide für das erfindungsgemäße Verfahren sind die beispielhaften Ausführungsformen (insbesondere das Ausführungsbeispiel 1) der WO 2004/108267. Auch können alle in diesen Schriften offenbarten Herstellverfahren zur Herstellung von erfindungsgemäß geeigneten Mo und V enthaltenden Multielementoxidmassen angewendet werden. Ebenso können die erfindungsgemäß erhältlichen Schalenkatalysatoren in allen in den vorgenannten Schriften offenbarten Verfahren der heterogen katalysierten partiellen Gasphasenoxidation von Acrolein zu Acrylsäure zur Gestaltung des Katalysatorfestbetts verwendet werden. Als Trägerkörper kommen für das erfindungsgemäße Verfahren grundsätzlich alle in den vorgenannten Schriften empfohlenen Trägerkörper in Betracht.

Der molare Anteil des Elementes Mo an der Gesamtmenge aller von Sauerstoff verschiedenen Elemente der erfindungsgemäß geeigneten feinteiligen Mo und V enthaltenden Multielementoxide beträgt in der Regel 5 bis 95 mol-%, häufig 10 bis 90 mol-% und vielfach 15 bis 85 mol-% bzw. 20 bis 80 mol-%. Das molare Verhältnis von Mo zu V beträgt in den für das erfindungsgemäße Verfahren geeigneten feinteiligen Mo und V enthaltenden Multielementoxidmassen in der Regel 15:1 bis 1:1, häufig 12:1 bis 2:1.

Neben Mo, V und O enthalten erfindungsgemäß geeignete feinteilige Multielementoxide häufig noch wenigstens eines der Elemente Nb und W. In vielen Fällen beträgt das molare Verhältnis Mo/(Gesamtmenge aus W und Nb) in solchen Multielementoxiden 80:1 bis 1:4. Häufig enthalten solche erfindungsgemäß geeigneten Multielementoxidmassen noch Cu im entsprechenden molaren Verhältnis Mo/Cu von 30:1 bis 1:3. Neben den Elementen Nb und/oder W sowie Mo, V, O und gegebenenfalls Cu, können für das erfindungsgemäße Verfahren geeignete feinteilige Multielementoxide zusätzlich z. B. wenigstens eines der Elemente Ta, Cr, Ce, Ni, Co, Fe, Mn, Zn, Sb, Bi, Alkali (Li, Na, K, Rb, Cs), H, Erdalkali (Mg, Ca, Sr, Ba), Si, Al, Ti und Zr enthalten. Natürlich kann die erfindungsgemäß zu verwendende feinteilige Multielementoxidmasse aber auch nur aus den Elementen Nb und/oder W, sowie Mo, V, O und gegebenenfalls Cu bestehen.

Erfindungsgemäß zu verwendende feinteilige Mo und V enthaltende Multielementoxide sind grundsätzlich dadurch erhältlich, dass man von Ausgangsverbindungen, die die von Sauerstoff verschiedenen elementaren Konstituenten der Multielementoxidmasse als Bestandteile enthalten, ein inniges Trockengemisch (auch Vorläufermasse genannt) herstellt und dieses bei Temperaturen von 200 bis 600 °C, vorzugsweise 300 bis 450 °C (Materialguttemperatur) thermisch behandelt (calciniert). Vorzugsweise erfolgt die thermische Behandlung in einer $O_2$ und $NH_3$ aufweisenden Gasatmosphäre. Das $NH_3$ kann sich dabei aus der Vorläufermasse selbst heraus entwickeln, in dem in selbige eine entsprechende Menge an Ammoniumionen eingearbeitet wird. Besonders bevorzugt (vgl. EP-A 72448, WO 2004/108267 und WO 95/11081) erfolgt die thermische Behandlung so, dass die Gasatmosphäre in der die thermische Behandlung erfolgt,

-   zu jedem Zeitpunkt 0,5 bis 4 Vol.-% $O_2$,
-   über die Gesamtdauer der thermischen Behandlung gemittelt 1 bis 8 Vol.-% $NH_3$, sowie
-   Wasserdampf und/oder Inertgas als Restmenge

enthält, wobei der $NH_3$-Gehalt der Atmospäre während der thermischen Behandlung ein Maximum durchläuft, das unterhalb von 20 Vol.-% liegt.

Eine Teilmenge von erfindungsgemäß günstigen, Mo und V enthaltenden, Multielementoxidmassen genügt der nachfolgenden allgemeinen Stöchiometrie I,

$$Mo_{12}V_aX^1_bX^2_cX^3_dX^4_eX^5_fX^6_gO_n \qquad (I),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = einer oder mehrere Alkalimetalle (Li, Na, K, Rb, Cs) und/oder H,
$X^5$ = eines oder mehrere Erdalkalimetalle (Mg, Ca, Sr, Ba),
$X^6$ = Si, Al, Ti und/oder Zr,
$a$ = 1 bis 6,
$b$ = 0,2 bis 4,
$c$ = 0 bis 18, vorzugsweise 0,5 bis 18,
$d$ = 0 bis 40,
$e$ = 0 bis 2,
$f$ = 0 bis 4,
$g$ = 0 bis 40 und
$n$ = eine Zahl, die durch die Wertigkeit und Häufigkeit der von Sauerstoff verschiedenen Elemente in I bestimmt wird.

Unter den erfindungsgemäß geeigneten feinteiligen Multielementoxidmassen (I) sind wiederum jene bevorzugt, bei denen die Variablen in den folgenden Bereichen liegen:

$X^1$ = W, Nb und/oder Cr,

$X^2$ = Cu, Ni, Co und/oder Fe,
$X^3$ = Sb,
$X^4$ = Na und/oder K,
$X^5$ = Ca, Sr und/oder Ba,
$X^6$ = Si, Al und/oder Ti,
a = 2,5 bis 5,
b = 0,5 bis 2,
c = 0,5 bis 3,
d = 0 bis 2,
e = 0 bis 0,2,
f = 0 bis 1,
g = 0 bis 15 und
n = eine Zahl, die durch die Wertigkeit und Häufigkeit der von Sauerstoff verschiedenen Elemente in I bestimmt wird.

Ganz besonders bevorzugte erfindungsgemäß geeignete Multielementoxidaktivmassen genügen der allgemeinen Stöchiometrie II,

$$Mo_{12}V_aX^1{}_bX^2{}_cX^5{}_fX^6{}_gO_n \qquad (II),$$

in der die Variablen folgende Bedeutung haben:

$X^1$ = W und/oder Nb,
$X^2$ = Cu und/oder Ni,
$X^5$ = Co und/oder Sr,
$X^6$ = Si und/oder Al,
a = 3 bis 4,5,
b = 1 bis 1,5,
c = 0,75 bis 2,5,
f = 0 bis 0,5,
g = 0 bis 8 und
n = eine Zahl, die durch die Wertigkeit und Häufigkeit der von Sauerstoff verschiedenen Elemente in II bestimmt wird.

Zur Herstellung von solchen und anderen erfindungsgemäß geeigneten feinteiligen Multielementoxidmassen geht man, wie bereits gesagt, von in an sich bekannter Weise geeigneten Quellen (Ausgangsverbindungen) der von Sauerstoff verschiedenen elementaren Konstituenten der gewünschten Multielementoxidmasse in im in der Multielementoxidmasse angestrebten jeweiligen stöchiometrischen Verhältnis aus, und erzeugt aus diesen ein möglichst inniges, vorzugsweise feinteiliges, Trockengemisch, welches dann der thermischen Behandlung unterworfen wird. Dabei kann es sich bei den Quellen entweder bereits um Oxide handeln, oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht.

Geeignete Ausgangsverbindungen des Mo, V, W und Nb sind auch deren Oxoverbindungen (Molybdate, Vanadate, Wolframate und Niobate) bzw. die von diesen abgeleiteten Säuren. Sauerstoff haltige Quellen sind ebenfalls günstig.

Ein wie bereits beschrieben vorteilhafter Gehalt des innigen Trockengemischs an Ammoniumionen kann in einfacher Weise dadurch realisiert werden, dass man in das innige Trockengemisch eine entsprechende Menge Ammoniumionen einarbeitet. Zweckmäßigerweise lassen sich die Ammoniumionen in das innige Trockengemisch z.B. dadurch einbringen, dass man als Quellen der Elemente Mo, V, W oder Nb die entsprechenden Ammoniumoxometallate verwendet. Beispiele hierfür sind Ammoniummetaniobat, Ammoniummetavanadat, Ammoniumheptamolybdattetrahydrat und Ammoniumparawolframatheptahydrat. Selbstverständlich können in das thermisch zu behandelnde innige Trockengemisch aber auch unabhängig von den als Quellen der Multielementoxidmassenkonstituenten erforderlichen Ausgangsverbindungen Ammoniumlieferanten wie $NH_4NO_3$, oder $NH_4Cl$, oder Ammoniumacetat, oder Ammoniumcarbonat, oder Ammoniumhydrogencarbonat, oder $NH_4OH$, oder $NH_4CHO_2$, oder Ammoniumoxalat eingearbeitet werden.

Das innige Vermischen der Ausgangsverbindungen kann prinzipiell in trockener oder in nasser Form erfolgen.

Vorzugsweise erfolgt das innige Vermischen in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen und Ausgangsverbindungen ausgegangen wird. Als Lösungsmittel wird bevorzugt Wasser eingesetzt. Anschließend wird die wässrige Masse (Lösung oder Suspension) getrocknet und das so erhaltene innige Trockengemisch gegebenenfalls unmittelbar thermisch behandelt. Vorzugsweise erfolgt der Trocknungsprozess durch Sprühtrocknung (die Austrittstemperaturen betragen in der Regel 100 bis 150°C) und unmittelbar im Anschluss an die Fertigstellung der wässrigen Lösung oder Suspension. Das dabei anfallende Pulver erweist sich für eine unmittelbare Weiterverarbeitung häufig als zu feinteilig, weshalb es dann unter Zusatz von z. B. Wasser zweckmäßigerweise geknetet wird. Vielfach erweist sich beim Kneten ein Zusatz einer niederen organischen Carbonsäure (z. B. Essigsäure) als vorteilhaft (typische Zusatzmengen liegen bei 5 bis 10 Gew.-% bezogen auf eingesetzte Pulvermasse).

Die anfallende Knetmasse wird anschließend anwendungstechnisch zweckmäßig zu Strānglingen geformt, diese werden wie bereits beschrieben thermisch behandelt und danach zu einem feinteiligen Pulver vermahlen, welches als solches oder auf eine gewünschte Korngröße eingeengt im erfindungsgemäßen Verfahren eingesetzt werden kann.

Für erfindungsgemäß erhältliche Schalenkatalysatoren geeignete Trägermaterialien sind z.B. poröse oder unporöse (bevorzugt) Aluminiumoxide, Siliciumdioxid, Thoriumdioxid, Zirkondioxid, Siliciumcarbid oder Silikate wie Magnesium- oder Aluminiumsilicat (z.B. Steatit des Typs C 220 der Fa. CeramTec). Die Materialien der Trägerkörper sind vorzugsweise chemisch inert, d. h., sie greifen in den Ablauf der Gasphasenpartialoxidation, die durch die erfindungsgemäß hergestellten Schalenkatalysatoren katalysiert wird, im wesentlichen nicht ein.

Die Trägerkörper können regelmäßig oder unregelmäßig geformt sein, wobei regelmäßig geformte Trägerkörper mit deutlich ausgebildeter Oberflächenrauhigkeit, z. B. Kugeln, Zylinder oder Hohlzylinder mit Splittauflage, bevorzugt werden. Ihre Längstausdehnung beträgt in der Regel 1 bis 10 mm.
Die Trägermaterialien können porös oder unporös sein. Vorzugsweise ist das Trägermaterial unporös (Gesamtvolumen der Poren auf das Volumen des Trägerkörpers bezogen vorteilhaft $\leq$ 1 Vol.-%).

Eine erhöhte Oberflächenrauhigkeit des Trägerkörpers bedingt in der Regel eine erhöhte Haftfestigkeit der aufgebrachten Schale aus feinteiliger Aktivmasse.

Vorzugsweise liegt die Oberflächenrauhigkeit Rz des Trägerkörpers im Bereich von 30 bis 100 $\mu$m, vorzugsweise 50 bis 70 $\mu$m (bestimmt gemäß DIN 4768 Blatt 1 mit einem "Hommel Tester für DIN-ISO-Oberflächenmeßgrößen" der Fa. Hommelwerke). Besonders bevorzugt sind oberflächenrauhe Trägerkörper der Fa. CeramTec aus Steatit C 220.

Erfindungsgemäß besonders geeignet ist die Verwendung von im wesentlichen unporösen, oberflächenrauhen, kugelförmigen Trägern aus Steatit (z. B. Steatit des Typs C 220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper.

Das erfindungsgemäß auf die Oberfläche des Trägerkörpers aufzubringende feinteilige Gemisch aus wenigsten einem die Elemente Mo und V enthaltenden feinteiligen Multielementoxid und wenigstens einer feinteiligen Substanz S sollte eine möglichst homogen gestaltete Mischung sein. Um aus den feinteiligem Ausgangsmaterialien ein solchermaßen homogenes Gemisch herzustellen, kann z. B. ein Mischer vom Typ R 645 der Fa. AMK in Aachen (DE) verwendet werden. Dabei handelt es sich um einen Schräglagemischer mit Schneidflügel (Intensivmischer). Der Mischarm dreht sich z. B. mit 39 Umdrehungen pro Minute und der Schneidflügel mit 3000 Umdrehungen pro Minute. Selbstredend können aber auch andere Mischer verwendet werden. Beispielsweise kann auch ein Eirich-Intensivmischer (Typ R 02) der Maschinenfabrik Gustav Eirich GmbH & Co. KG, D-74736 Hardheim verwendet werden.

Die (Schalen)Dicke $D_A$ der beim erfindungsgemäßen Verfahren auf den Trägerkörper aufgebrachten aktiven Masse liegt in der Regel zweckmäßigerweise bei 10 bis 1000 $\mu$m. Bevorzugt sind, insbesondere bei ringförmigen Trägerkörpern, 10 bis 500 $\mu$m, besonders bevorzugt 100 bis 500 $\mu$m und ganz besonders bevorzugt 200 bis 300 bzw. 150 bis 250 $\mu$m.

Die Körnung (Feinheit) des Mo und V enthaltenden feinteiligen Multielementoxids wird natürlich in gleicher Weise wie die Körnung der feinteiligen Substanz S anwendungstechnisch zweckmäßig an die angestrebte Schalendicke $D_A$ angepasst. Alle bezüglich der Längstausdehnung $d_L$ der feinteiligen Substanz S gemachten Aussagen gelten daher in entsprechender Weise für die Längstausdehnung $d_L$ des feinteiligen Mo und V enthaltenden Multielementoxids.

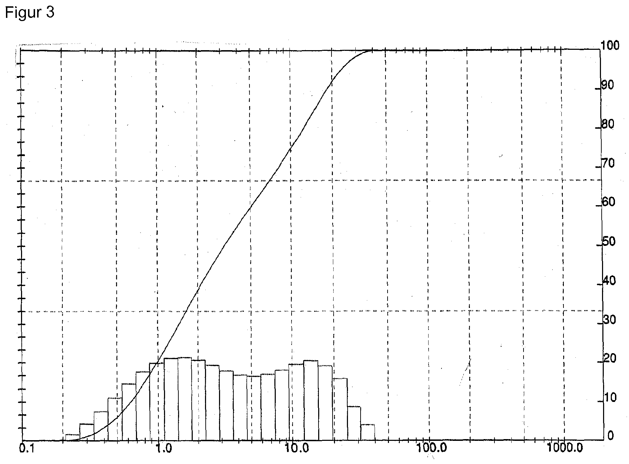

Für den Vorzugsbereich einer Schalendicke $D_A$ von 100 bis 500 $\mu$m eignen sich insbesondere solche feinteiligen Mo und V enthaltenden Multielementoxide, von denen $\geq$ 50 %, vorzugsweise $\geq$ 75 % des Gesamtvolumens aller Partikel ein Sieb der Maschenweite 1 bis 20 $\mu$m, vorzugsweise 1 bis 10 $\mu$m passieren und deren Anteil an Partikeln mit einer Längstausdehnung $d_L$ oberhalb von 50 $\mu$m und unterhalb von 0,2 $\mu$m weniger als 1 % des Gesamtvolumen aller Partikel beträgt. In der Regel entspricht die Verteilung der Längstausdehnung $d_L$ sowohl der feinteiligen Substanz S als auch des feinteiligen Mo und V enthaltenden Multielementoxids herstellungsbedingt einer Gaußverteilung. Erfindungsgemäß ganz besonders bevorzugt weist das feinteilige Mo und V enthaltende Multielementoxid für den Schalendicke $D_A$-Bereich von 100 bis 500 $\mu$m die in Figur 3 gezeigte Partikeldurchmesserverteilung auf. Die Abszisse zeigt die Durchmesser [$\mu$m] im logarithmischen Maßstab. Die Ordinate zeigt den Volumenanteil, der den jeweiligen Durchmesser oder einen kleineren Durchmesser aufweist.
Das Anheften (Aufbringen) der feinteiligen aktiven Masse auf die Oberfläche des Trägerkörpers kann beim erfindungsgemäßen Verfahren entsprechend den im Stand der Technik diesbezüglich beschriebenen Verfahren erfolgen (vgl. z. B. US-A 2006/0205978 sowie EP-A 714700 und den in diesen beiden Schriften zitierten Stand der Technik).

Erfindungsgemäß zweckmäßig wird man das Aufbringen der aktiven Masse auf die Oberfläche des Trägerkörpers mit Hilfe eines flüssigen Bindemittels vornehmen. Als ein solches flüssiges Bindemittel kommt z. B. Wasser, ein organisches Lösungsmittel oder eine Lösung einer organischen Substanz (z. B. eines organischen Lösungsmittels) in Wasser oder in einem organischen Lösungsmittel in Betracht.

Beispielhaft genannt seien als organische Bindemittel ein- oder mehrwertige organische Alkohole wie z. B. Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Als in Wasser, in einer organischen Flüssigkeit oder in einem Gemisch aus Wasser und einer organischen Flüssigkeit lösliche organische Bindemittelpromotoren sind z. B. Monosaccharide und Oligosaccharide wie Glucose, Fructase, Saccharose und/oder Lactose geeignet.

Besonders vorteilhaft wird als flüssiges Bindemittel eine Lösung bestehend aus 20 bis 90 Gew.-% Wasser und 10 bis 80 Gew.-% einer organischen Verbindung verwendet. Vorzugsweise beträgt der organische Anteil an den vorgenannten flüssigen Bindemitteln 10 bis 50 und besonders bevorzugt 20 bis 30 Gew.-%.

Bevorzugt sind generell solche organischen Bindemittel bzw. Bindemittelanteile, deren Siedpunkt oder Sublimationstemperatur bei Normaldruck (1 atm) $\geq$ 100 °C, vorzugsweise $\geq$ 150 °C beträgt. Ganz besonders bevorzugt liegt der Siedepunkt oder Sublimationspunkt solcher organischen Bindemittel bzw. Bindemittelanteile bei Normaldruck gleichzeitig unterhalb der im Rahmen der Herstellung des die Elemente Mo und V enthaltenden feinteiligen Multielementoxids angewandten höchsten (Calcinations) Temperatur. Üblicherweise liegt diese höchste Calcinationstemperatur bei $\leq$ 600 °C, häufig bei $\leq$ 500 °C oder bei $\leq$ 400 °C, vielfach sogar bei $\leq$ 300 °C. Besonders bevorzugte flüssige Bindemittel sind Lösungen, die aus 20 bis 90 Gew.-% Wasser und 10 bis 80 Gew.-% Glycerin bestehen. Vorzugsweise beträgt der Glycerinanteil in diesen wässrigen Lösungen 10 bis 50 und besonders bevorzugt 20 bis 30 Gew.-%. Die Vorteilhaftigkeit von erfindungsgemäß bevorzugten Bindemitteln liegt unter anderem darin begründet, dass sie sowohl die feinteilige aktive Masse als auch die Trägerkörper in voll befriedigender Weise zu benetzen vermögen.

Das Aufbringen (Anheften) des feinteiligen Gemischs aus wenigstens einem die Elemente Mo und V enthaltenden feinteiligen Multielementoxid und wenigstens einer feinteiligen Substanz S kann in besonders einfacher Weise dadurch erfolgen, dass man das feinteilige Gemisch in dem flüssigen Bindemittel dispers verteilt und die dabei resultierende Suspension auf bewegte und gegebenenfalls heiße Trägerkörper aufsprüht (vgl. z. B. DE-A 1642921, DE-A 2106796 und die DE-A 2626887).

Nach Beendigung des Aufsprühens kann der Lehre der DE-A 2909670 folgend durch Überleiten von heißer Luft der Feuchtigkeitsgehalt der resultierenden Schalenkatalysatoren gemindert werden. Hinsichtlich der in einem erfindungsgemäß hergestellten Schalenkatalysator verbleibenden Restfeuchte wird man sich anwendungstechnisch zweckmäßig an der Lehre der DE-A 102005010645 orientieren.

Erfindungsgemäß bevorzugt wird man das Anheften der feinteiligen aktiven Masse auf die Trägerkörperoberfläche im Rahmen des erfindungsgemäßen Verfahrens jedoch so vornehmen (vgl. DE-A 2526238, US-A 3956377, DE-A 235151, DE-A 2909671 und EP-A 714700), dass man die Trägerkörper zunächst mit dem flüssigen Bindemittel befeuchtet und nachfolgend feinteilige aktive Masse dadurch an das mit Bindemittel angefeuchtete Trägermaterial anheftet (auf dessen Oberfläche aufbringt), dass man das befeuchtete Trägermaterial in der feinteiligen aktiven Masse wälzt.

Zur Erzielung der gewünschten Schalendicke wird das vorbeschriebene Verfahren vorteilhaft periodisch wiederholt. D. h., der grundbeschichtete Trägerkörper bildet dann den in der darauffolgenden Periode zunächst zu befeuchtenden und dann durch Kontakt mit trockener feinteiliger aktiver Masse zu beschichtenden Trägerkörper.

Für eine Durchführung des erfindungsgemäßen Verfahrens im technischen Maßstab empfiehlt sich daher vorteilhaft die Anwendung des in der DE-A 2909671 offenbarten Verfahrensprinzips, jedoch vorzugsweise unter Verwendung der in der EP-A 714700 empfohlenen Bindemittel.

D. h., die zu beschichtenden Trägerkörper werden in einen vorzugsweise geneigten (der Neigungswinke beträgt in der Regel 30 bis 90°) rotierenden Drehbehälter (z. B. Drehteller oder Dragierkessel) gefüllt. Der rotierende Drehbehälter führt die insbesondere kugelförmigen oder zylindrischen, vor allem hohlzylindrischen, Trägerkörper unter zwei in bestimmtem Abstand aufeinanderfolgend angeordneten Dosiervorrichtungen hindurch. Die erste der beiden Dosiervorrichtungen entspricht zweckmäßig einer Düse, durch die die im rotierenden Drehteller rollenden Trägerkörper mit dem zu verwendenden flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige aktive Masse zuzuführen (z. B. über eine Schüttelrinne). Die kontrolliert befeuchteten Trägerkugeln nehmen das zugeführte Aktivmassenpulver auf, das sich durch die rollende Bewegung auf der äußeren Oberfläche der zylinder- oder kugelförmigen Trägerkörper zu einer zusammenhängenden Schale verdichtet (im inneren Kreis eines hohlzylinderischen Trägerkörpers findet eine solche verdichtende Bewegung nicht statt, weshalb dieser im wesentlichen unbeschichtet bleibt).

Bei Bedarf durchläuft der so grundbeschichtete Trägerkörper im Verlauf der darauffolgenden Umdrehung wiederum die Sprühdüse, wird dabei kontrolliert befeuchtet, um im Verlauf der Weiterbewegung eine weitere Schicht feinteiliger oxidischer Aktivmasse aufnehmen zu können u.s.w.. (eine Zwischentrocknung ist in der Regel nicht erforderlich). Die Entfernung des erfindungsgemäß verwendeten flüssigen Bindemittels kann, sich an der Lehre der DE-A 102005010645 orientierend, teilweise oder vollständig z. B. durch abschließende Wärmezufuhr, z. B. durch Einwirkung von heißen Gasen, wie $N_2$ oder Luft, erfolgen. Ein besonderer Vorzug der vorstehend beschriebenen Ausführungsform des erfindungemäßen Verfahrens besteht darin, dass in einem Arbeitsgang Schalenkatalysatoren mit schichtförmig aus zwei oder mehr unterschiedlichen aktiven Massen bestehenden Schalen hergestellt werden können. Bemerkenswerterweise bewirkt das erfindungsgemäße Verfahren dabei sowohl eine voll befriedigende Haftung der aufeinanderfolgenden Schichten aneinander, als auch der Grundschicht auf der Oberfläche des Trägerkörpers. Dies gilt auch im Fall von ringförmigen Trägerkörpern. Wesentlich für die vorbeschriebene Ausführungsform des erfindungsgemäßen Verfahrens ist, dass die Befeuchtung der zu beschichtenden Oberfläche des Trägerkörpers in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige aktive Masse zu getrennten Agglomeraten, anstatt auf die Oberfläche aufzuziehen. Detailliertere Angaben hierzu finden sich in der DE-A 2909671.

Ein Vorzug des vorstehend beschriebenen Verfahrens besteht darin, dass die Entfernung des verwendeten flüssigen Bindemittels in kontrollierter Weise z. B. durch Verdampfen und/oder Sublimieren vorgenommen werden kann. Im einfachsten Fall kann dies durch die Einwirkung heißer Gase entsprechender Temperatur (z. B. 50 bis 200 °C, häufig 100 bis 150 °C) erfolgen. Durch die Einwirkung heißer Gase kann der Lehre der DE-A 102005010645 folgend aber auch nur eine Teiltrocknung bewirkt werden. Die Endtrocknung kann dann beispielsweise im Reaktor für die relevante Partialoxidation selbst befindlich erfolgen (natürlich könnte eine Nachfolgetrocknung bis zur Volltrocknung auch in einem Trockenofen beliebiger Art (z. B. in einem Bandtrockner) erfolgen. Grundsätzlich sollte die im Rahmen der Trocknung einwirkende Temperatur erfindungsgemäß vorteilhaft nicht oberhalb der zur Herstellung der die Elemente Mo und V enthaltenden feinteiligen Multielementoxidmasse angewendeten Calcinationstemperatur liegen.

An dieser Stelle sei noch festgehalten, dass sich für das erfindungsgemäße Verfahren auch feinteilige Multielementoxidmassen eignen, die als von Sauerstoff verschiedene Elemente neben den Elementen Mo und V wenigstens eines der beiden Elemente Te und Sb, und wenigstens eines der Elemente aus der Gruppe umfassend Nb, Pb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthalten.

Ihre Herstellung kann z. B. wie auf Seiten 25, 26 der WO 2004/108267 beschrieben erfolgen. Bevorzugt enthält die Kombination dabei aus der letzten Elementgruppe die Elemente Nb, Ta, W und/oder Ti und besonders bevorzugt das Element Nb.

Bevorzugt enthalten die vorgenannten feinteiligen Multielementoxidmassen die vorgenannte Elementkombination in der Stöchiometrie III

$$Mo_1V_bM^1{}_cM^2{}_d \qquad \text{(III)},$$

mit

M$^1$ = Te und/oder Sb,

M$^2$ = wenigstens eines der Elemente aus der Gruppe umfassen Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au und In,

b = 0,01 bis 1,

c = > 0 bis 1, und

d = > 0 bis 1.

Bevorzugt ist M$^1$ = Te und M$^2$ = Nb, Ta, W und/oder Ti. Vorzugsweise ist M$^2$ = Nb.

Der stöchiometrische Koeffizient b beträgt mit Vorteil 0,1 bis 0,6. In entsprechender Weise beläuft sich der Vorzugsbereich für den stöchiometrischen Koeffizienten c auf 0,01 bis 1 bzw. auf 0,05 bis 0,4 und günstige Werte für d betragen 0,01 bis 1 bzw. 0,1 bis 0,6.

Besonders günstig ist es, wenn die stöchiometrischen Koeffizienten b, c und d simultan in den vorgenannten Vorzugsbereichen liegen.

Das Vorgenannte gilt insbesondere dann, wenn die Aktivmasse hinsichtlich ihrer von Sauerstoff verschiedenen Elemente aus einer vorgenannten Elementkombination besteht.

Dies sind dann insbesondere die Multielementoxidaktivmassen der allgemeinen Stöchiometrie IV

$$Mo_1V_bM^1{}_cM^2{}_dO_n \qquad \text{(IV)},$$

wobei die Variablen die bezüglich der Stöchiometrie III angeführte Bedeutung aufweisen und n = eine Zahl ist, die durch die Wertigkeit und Häufigkeit der von Sauerstoff verschiedenen Elemente in (IV) bestimmt wird.

Ferner eignen sich für das erfindungsgemäße Verfahren solche Multielementoxidmassen, die einerseits entweder eine der vorgenannten Elementkombinationen enthalten oder, bezüglich der von Sauerstoff verschiedenen Elemente, aus ihr bestehen und gleichzeitig ein Röntgendiffraktogramm aufweisen, das Beugungsreflexe h und i zeigt, deren Scheitelpunkte bei den Beugungswinkeln (2Θ) 22,2 + 0,5° (h) und 27,3 + 0,5° (i) liegen (alle in dieser Schrift auf ein Röntgendiffraktogramm bezogenen Angaben beziehen sich auf ein unter Anwendung von Cu-Kα-Strahlung als Röntgenstrahlung erzeugtes Röntgendiffraktogramm (Siemens-Diffraktometer Theta-Theta D-5000, Röhrenspannung : 40 kV, Röhrenstrom : 40mA, Aperturblende V20 (variabel), Streustrahlblende V20 (variabel), Sekundärmonochromatorblende (0,1 mm), Detektorblende (0,6 mm), Meßintervall (2Θ) : 0,02°, Meßzeit je Schritt: 2,4 s, Detektor: Scintillationszählrohr)).

Die Halbwertsbreite dieser Beugungsreflexe kann dabei sehr klein oder auch sehr ausgeprägt sein.

Besonders eignen sich für das erfindungsgemäße Verfahren diejenigen der vorgenannten Multielementoxidmassen, deren Röntgendiffraktogramm zusätzlich zu den Beugungsreflexen h und i einen Beugungsreflex k aufweist, dessen Scheitelpunkt bei 28,2 + 0,5° (k) liegt.

Unter den letzteren sind für eine erfindungsgemäße Verwendung wiederum jene bevorzugt, bei denen der Beugungsreflex h innerhalb des Röntgendiffraktogramms der intensitätsstärkste ist, sowie eine Halbwertsbreite von höchstens 0,5° aufweist, und ganz besonders bevorzugt eignet sich das erfindungsgemäße Verfahren für jene, bei denen die Halbwertsbreite des Beugungsreflexes i und des Beugungsreflexes k gleichzeitig jeweils ≤ 1° beträgt und die Intensität $P_k$ des Beugungsreflexes k und die Intensität $P_i$ des Beugungsreflexes i die Beziehung $0,2 \leq R \leq 0,85$, besser $0,3 \leq R \leq 0,85$, bevorzugt $0,4 \leq R \leq 0,85$, besonders bevorzugt $0,65 \leq R \leq 0,85$, noch mehr bevorzugt $0,67 \leq R \leq 0,75$ und ganz besonders bevorzugt R = 0,70 bis 0,75 bzw. R = 0,72 erfüllen, in der R das durch die Formel

$$R = P_i/(P_i+P_k)$$

definierte Intensitätsverhältnis ist. Bevorzugt weisen die vorgenannten Röntgendiffraktogramme keinen Beugungsreflex auf, dessen Maximum bei 2Θ = 50 + 0,3° liegt.

Die Definition der Intensität eines Beugungsreflexes im Röntgendiffraktogramm bezieht sich in dieser Schrift auf die in der DE-A 19835247, der DE-A 10122027, sowie die in der DE-A 10051419 und DE-A 10046672 niedergelegte Definition. Das gleiche gilt für die Definition der Halbwertsbreite.

Neben den Beugungsreflexen h, i und k enthalten die vorgenannten Röntgendiffraktogramme von erfindungsgemäß vorteilhaft zu verwendenden Multielementoxidaktivmassen noch weitere Beugungsreflexe, deren Scheitepunkte bei den nachfolgenden Beugungswinkeln (2Θ) liegen:

$$9,0 \pm 0,4° \text{ (l)}$$

$$6,7 \pm 0,4° \text{ (o)}$$

und

$$7,9 \pm 0,4° \text{ (p)}.$$

Günstig ist es ferner, wenn das Röntgendiffraktogramm zusätzlich einen Beugungsreflex enthält, dessen Scheitelpunkt beim Beugungswinkel (2Θ) = 45,2 + 0,4° (q) liegt.

Häufig enthält das Röntgendiffraktogramm auch noch die Reflexe 29,2 + 0,4° (m) und 35,4 ± 0,4° (n).

Es ist ferner günstig, wenn die in den Formeln III und IV definierten Elementkombinationen als reine i-Phase vorliegen. Enthält die Multielementoxidmasse auch noch k-Phase, enthält ihr Röntgendiffraktogramm neben den oben genannten noch weitere Beugungsreflexe, deren Scheitelpunkte bei den nachfolgenden Beugungswinkeln (2Θ) liegen: 36,2 + 0,4° (m) und 50 + 0,4° (die Begriffe i- und k-Phase werden in dieser Schrift wie in der DE-A 10122027 und DE-A 10119933 festgelegt verwendet).

Ordnet man dem Beugungsreflex h die Intensität 100 zu, ist es günstig, wenn die Beugungsreflexe i, l, m, n, o, p, q in der gleichen Intensitätsskala die nachfolgenden Intensitäten aufweisen:

| | |
|---|---|
| i: | 5 bis 95, häufig 5 bis 80, teilweise 10 bis 60; |
| l: | 1 bis 30; |
| m: | 1 bis 40; |
| n: | 1 bis 40; |
| o: | 1 bis 30; |
| p: | 1 bis 30 und |
| q: | 5 bis 60. |

Enthält das Röntgendiffraktogramm von den vorgenannten zusätzlichen Beugungsreflexen, ist die Halbwertsbreite derselben in der Regel ≤ 1°.

Die spezifische Oberfläche von erfindungsgemäß zu verwendeten Multielementoxidmassen der allgemeinen Formel IV oder von Multielementoxidmassen, die Elementkombinationen der allgemeinen Formel III enthalten, beträgt vielfach 1 bis 30 $m^2/g$ (BET-Oberfläche, Stickstoff), vor allem dann, wenn ihr Röntgendiffraktogramm wie beschrieben gestaltet ist.

Wie bereits erwähnt, eignen sich die erfindungsgemäß erhältlichen Schalenkatalysatoren insbesondere zur Durchführung der heterogen katalysierten partiellen Gasphasenoxidation von Acrolein zu Acrylsäure. Sie zeichnen sich dabei insbesondere dadurch aus, dass ein mit ihnen beschicktes Katalysatorfestbett bei der Durchführung der vorgenannten Partialoxidation eine erhöhte Standzeit aufweist.

Dies gilt vor allem dann, wenn die heterogen katalysierte partielle Gasphasenoxidation von Acrolein zu Acrylsäure bei hohen Acroleinlasten durchgeführt wird, wie es z. B. die DE-A 10307983, die DE-A 19948523 und die DE-A 19910508 beschreiben.

In der Regel wird die Gasphasenpartialoxidation des Acroleins dabei in einem eine oder mehrere Temperaturzonen aufweisenden Rohrbündelreaktor durchgeführt wie es z.B. die EP-A 700714, die EP-A700 893, die DE-A 19910508, die DE-A 19948523, die DE-A 19910506, die DE-A 19948241, die DE-C 2830765, die DE-C 2513405, die US-A 3147084, die DE-A 2201528, die EP-A 383224 und die DE-A 2903218 beschreiben.

Die Feststoffkatalysatorschüttung befindet sich dabei in den Metallrohren (Kontaktrohren) des Rohrbündelreaktors und um die Metallrohre werden das oder die Temperiermedien geführt (bei mehr als einer Temperaturzone wird eine entsprechende Anzahl räumlich getrennter Temperiermedien um die Metallrohre geführt). Das Temperiermedium ist in der Regel eine Salzschmelze. Durch die Kontaktrohre wird das Reaktionsgasgemisch geführt.

Die Festbettkatalysatorschüttung kann nur aus erfindungsgemäß erhältlichen Katalysatoren, aber auch aus solchen Katalysatoren mit inerten Formkörper verdünnt bestehen. Als inerte Formkörper können dabei z. B. die zur Herstellung erfindungsgemäßer Schalenkatalysatoren verwendeten Trägerformkörper (Trägerkörper) eingesetzt werden. Vor und/oder hinter der Festbettkatalysatorschüttung kann sich eine reine Inertformkörperschüttung befinden (derartige reine Inertformkörperschüttungen werden bei der Berechnung der Belastung des Katalysatorfestbetts mit Reaktionsgas bzw. mit einer Reaktionsgaskomponente normalerweise nicht miteinbezogen).

Die Kontaktrohre sind üblicherweise aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m.

Anwendungstechnisch zweckmäßig beläuft sich die im Rohrbündelbehälter untergebrachte Anzahl an Kontaktrohren auf wenigstens 5000, vorzugsweise auf wenigstens 10 000. Häufig beträgt die Anzahl der im Reaktorbehälter untergebrachten Kontaktrohre 15 000 bis 40 000. Rohrbündelreaktoren mit einer oberhalb von 50 000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Eine Beschickung von Kontaktrohren in Rohrbündelreaktoren mit erfindungsgemäß erhältlichen Katalysatoren ist vor allem dann vorteilhaft, wenn der Rohrbündelreaktor bei einer Acrolein-Belastung der Katalysatorbeschickung betrieben wird, die ≥135 Nl/l·h, oder ≥150 Nl/l·h, oder ≥160 Nl/l·h, oder ≥170 Nl/l·h, oder >180 Nl/l·h, oder ≥200 Nl/l·h, oder ≥220 Nl/l·h, oder ≥240 Nl/l·h beträgt. Selbstredend ist eine solche Katalysatorbeschickung auch bei kleineren (z. B ≤ 130 Nl/l•h, oder ≤ 100 Nl/l•h, oder ≤ 80 Nl/l•h) Acroleinbelastungen vorteilhaft.

In der Regel wird die Acroleinbelastung der Katalysatorbeschickung jedoch ≤ 350 Nl/l•h, oder ≤ 300 Nl/l•h, oder ≤ 250 Nl/l•h betragen.

Die volumenspezfische Aktivität des Katalysatorfestbetts wird man dabei in der Regel so gestalten, dass sie in Strömungsrichtung des Reaktionsgases zunimmt.

Dies ist in einfacher Weise z. B. dadurch realisierbar, dass man in Strömungsrichtung des Reaktionsgases den Verdünnungsgrad der Katalysatorfestbetts mit inerten Formkörpern abnehmend gestaltet.

Im übrigen kann man die heterogen katalysierte Partialoxidation mit erfindungsgemäß erhältlichen Schalenkatalysatoren in allen Aspekten so durchführen, wie es die DE-A 10350822 ausführt. Der Acroleingehalt im Reaktionsgaseingangsgemisch kann z. B. bei Werten von 3 oder 6 bis 15 Vol.-%, häufig bei 4 oder 6 bis 10 Vol.-%, bzw. 5 bis 8 Vol.-% liegen (jeweils bezogen auf das Gesamtvolumen).

Das molare Verhältnis von $O_2$:Acrolein im Reaktionsgaseingangsgemisch wird normalerweise $\geq 1$ betragen. Üblicherweise wird dieses Verhältnis bei Werten $\leq 3$ liegen. Vielfach wird man die heterogen katalysierte Acroleinpartialoxidation zu Acrylsäure mit einem im Reaktionsgaseingangsgemisch vorliegenden Acrolein : Sauerstoff: Wasserdampf : Inertgas-Volumenverhältnis (NI) von 1 : (1bis 3) : (0 bis 20) : (3 bis 30), vorzugsweise von 1 : (1 bis 3) : (0,5 bis 10) : (7 bis 10) ausführen.

Als inerte Verdünnungsgase kommen dabei u. a. $N_2$, $CO_2$, CO, Edelgase, Propan, Ethan, Methan, Butan und/oder Pentan (d. h., jedes als alleiniges Verdünnungsgas oder im Gemisch mit einem anderen oder mit mehreren anderen dieser inerten Verdünnungsgase) in Betracht. Die Reaktionstemperaturen einer solchen heterogen katalysierten Acroleinpartialoxidation liegen dabei üblicherweise im Bereich von 200 bis 380 °C, in der Regel 220 bis 350 °C, häufig 245 bis 285 °C bzw. 245 bis 265 °C. Der Arbeitsdruck beträgt normalerweise 1 bis 3 bar.

Der Acroleinumsatz, bezogen auf einmaligen Durchgang des Reaktionsgasgemischs durch das Katalysatorfestbett, beträgt üblicherweise $\geq 96$ mol-%, häufig $\geq 98$ mol-%, und vielfach $\geq 99$ mol-%.

Zusammenfassend beinhaltet die vorliegende Anmeldung insbesondere (Schalen) Katalysatoren , die aus einem Trägerkörper und einer auf die Oberfläche des Trägerkörpers aufgebrachten katalytisch aktiven Masse sowie gegebenenfalls Bindemittel bestehen, wobei die katalytisch aktive Masse ein feinteiliges Gemisch aus

- wenigstens einem die Elemente Mo und V enthaltenden feinteiligen Multielementoxid und
- wenigstens einer feinteiligen Substanz S ausgewählt aus der Gruppe bestehend aus Oxiden des Molybdäns und aus Verbindungen des Molybdäns, aus denen sich unter der Einwirkung von erhöhter Temperatur und molekularem Sauerstoff ein Oxid des Molybdäns bildet,

ist.

Schalenkatalysatoren der erfindungsgemäßen Art eignen sich insbesondere bei heterogen katalysierten Partialoxidationen von Acrolein zu Acrylsäure durch eine erhöhte Standzeit aus. Grundsätzlich eignen sie sich aber auch als Katalysatoren für alle anderen in der EP-A 714700, der DE-A 10350822 sowie der WO 2004/108267 aufgeführten heterogen katalysierten Partialoxidationen als Katalysatoren mit verlängerter Standzeit. Katalysatoren, die nach Verfahren der vorliegenden Erfindung erhältlich sind, eignen sich insbesondere für alle in der US 2006/0161019 beschriebenen Partialoxidations- und Regenerierverfahren (insbesondere für die dort beschriebenen Partialoxidationen von Acrolein zu Acrylsäure).

Beispiel und Vergleichsbeispiel

1. Vergleichsbeispiel

Wie im Ausführungsbeispiel 1 der WO 2004/108267 beschrieben, wurde eine Multielementoxidmasse der Stöchiometrie $Mo_{12}V_3W_{1,2}Cu_{2,4}O_x$ hergestellt.

Das erhaltene katalytisch aktive Multielementoxid wurde mittels einer Biplexquerstromsichtmühle (BQ 500) (Fa. Hosokawa-Alpine Augsburg) zu einem feinteiligen Pulver gemahlen, das die in Figur 3 gezeigte Partikeldurchmesserverteilung aufwies.

Mittels des gemahlenen Pulvers wurden wie in Beispiel S1 der EP-A 714700 ringförmige Trägerkörper (7 mm Außendurchmesser, 3 mm Länge, 4 mm Innendurchmesser (7 x 3 x 4 mm), Steatit C 220, Fa. Ceram Tec, mit einer Oberflächenrauhigkeit Rz von 62 $\mu$m, Bezeichnung: "Steatitring 7 x 3 x 4 porös beschichtet") beschichtet. Bindemittel war wie in Beispiel S1 der EP-A 714700 beschrieben eine wässrige Lösung aus 75 Gew.-% Wasser und 25 Gew.-% Glycerin. Der Aktivmassenanteil der resultierenden Schalenkatalysatoren wurde jedoch im Unterschied zu vorgenanntem Beispiel S1 zu 20 Gew.-% (bezogen auf das Gesamtgewicht aus Trägerkörper und Aktivmasse) gewählt. Das Mengenverhältnis von Pulver und Bindemittel wurde proportional angepasst. Die Weitertrocknung erfolge jedoch nicht wie in Beispiel S1 beschrieben in einem Hordenofen bei 250 °C, sondern in einem Memmertumlufttrockenschrank bei 300 °C (2 h).

Es wurde so ein Vergleichsschalenkatalysator VS1 erhalten.

2. Beispiel

Es wurde wie im Vergleichsbeispiel verfahren. Das gemahlene $Mo_{12}V_3W_{1,2}Cu_{2,4}O_x$ Pulver (400 g) wurde jedoch mit, bezogen auf das Gewicht des gemahlenen $Mo_{12}V_3W_{1,2}Cu_{2,4}O_x$ Pulvers, 15 Gew.-% an feinteiligem $MoO_3$ (60 g) versetzt ($MoO_3$ ("Molybdenum Trioxide I") der Fa. H. C. Starck, Mo-Gehalt = 66,6 Gew.-%, $O_M$ = 1 m$^2$/g, Partikeldurchmesserverteilung entsprechend Figur 2.

Abschließend wurde das Gemisch in einem Multimixer der Fa. Rotor Lips AG, CH-3661 Uetendorf, vom Typ GT 550 auf Stufe 8 über einen Zeitraum von 1 Minute homogen durchmischt. Mit dem resultierenden feinteiligen Gemisch wurde analog zum Vergleichsbeispiel ein ringförmiger Schalenkatalysator hergestellt. Der Schalenanteil wurde jedoch zu 22,33 Gew.-% (bezogen auf das Gesamtgewicht aus Trägerkörper und Schale) gewählt. Das Mengenverhältnis von Pulver und Bindemittel wurde entsprechend angepasst. Es wurde ein Ausführungsbeispielsschalenkatalysator AS1 erhalten.

2. Testung der Schalenkatalysatoren VS1 und AS1

Die Schalenkatalysatoren wurden jeweils wie folgt in einem von einem Salzbad (Gemisch aus 53 Gew.-% Kaliumnitrat, 40 Gew.-% Natriumnitrit und 7 Gew.-% Natriumnitrat) umspülten Modellkontaktrohr getestet:

| Modellkontaktrohr: | V2A-Stahl, 2 mm Wandstärke, 26 mm Innendurchmesser, zentriert eine Thermohülse (zur Aufnahme eines Thermoelements) des Außendurchmessers 4 mm, Rohrlänge 320 cm. Nach einer Vorschüttung der Länge 20 cm aus inerten Ringen (Steatit C 220, 7 x 3 x 4 mm) wurden in Strömungsrichtung die ersten 100 cm mit einem Gemisch aus 70 Gew.-% des jeweiligen Schalenkatalysators und 30 Gew.-% ringförmigen inerten Trägerkörpern aus Steatit C 220 (7 mm Außendurchmesser, 3 mm Länge, 4 mm Innendurchmesser) beschickt. Die in Strömungsrichtung nachfolgenden 200 cm wurden mit dem jeweiligen Schalenkatalysator in unverdünnter Form beschickt. |
|---|---|

Das Reaktionsgasgemisch wies folgende Ausgangszusammensetzung auf:

| Acrolein | 4,4 Vol.-%, |
|---|---|
| $O_2$ | 5,4 Vol.-%, |
| $H_2O$ | 10,8 Vol.-%, |
| CO | 0,5 Vol.-%, |
| $CO_2$ | 0,9 Vol.-%, |
| Acrylsäure | 0,4 Vol.-%, |
| Propylen | 0,3 Vol.-%, und als Restmenge bis 100 Vol.-% $N_2$. |

Die Acroleinbelastung des Katalysatorfestbetts wurde jeweils zu 90 Nl/l•h eingestellt (unter der Belastung eines einen Reaktionsschritt katalysierenden Katalysatorfestbetts mit Reaktionsgas oder mit einer Reaktionsgaskomponente wird dabei die Menge an Reaktionsgas bzw. an Reaktionsgaskomponente in Normlitern (= Nl; das Volumen in Litern, das die entsprechende Reaktionsgas- bzw. Reaktionsgaskomponentenmenge bei Normalbedingungen, d. h., bei 25 °C und 1 bar, einnehmen würde) verstanden, die pro Stunde durch einen Liter Katalysatorfestbett geführt wird).

Die Temperatur des Salzbades wurde jeweils so eingestellt, dass der Umsatz des Acroleins, bezogen auf einen einmaligen Durchgang des Reaktionsgasgemisch durch das Katalysatorfestbett 99,6 mol-% betrug (die dazu erforderliche Anfangssalzbadtemperatur betrug unabhängig vom verwendeten Schalenkatalysator 261 °C). Die Eintrittstemperatur des Reaktionsgasgemischs war auf die jeweilige Salzbadtemperatur eingestellt.

Bei Verwendung von Schalenkatalysator AS1 war dazu über eine Laufzeit von 58 Tagen keine Erhöhung der Salzbadtemperatur erforderlich. Die Selektivität der Acrylsäurebildung lag über die gesamte Laufzeit bei 94,8 mol-%.

Bei Verwendung von Schalenkatalysator VS1 war bereits über eine Laufzeit von 54 Tagen eine Erhöhung der Salzbadtemperatur von 1 °C erforderlich, um die einhergehende Katalysatordeaktivierung auszugleichen und den Acroleinumsatz von 99,6 mol-% aufrechtzuerhalten.

**Patentansprüche**

**1.** Verfahren zur Herstellung eines Katalysators bestehend aus einem Trägerkörper und einer auf der Oberfläche des Trägerkörpers aufgebrachten katalytisch aktiven Masse, bei dem man die aktive Masse mit Hilfe eines Bindemittels an die Oberfläche des Trägerkörpers anheftet, **dadurch gekennzeichnet, dass** die aktive Masse ein feinteiliges Gemisch aus

- wenigstens einem die Elemente Mo und V enthaltenden feinteiligen Multielementoxid und
- wenigstens einer feinteiligen Substanz S ausgewählt aus der Gruppe bestehend aus Oxiden des Molybdäns und aus Verbindungen des Molybdäns, aus denen sich unter der Einwirkung von erhöhter Temperatur und molekularem Sauerstoff ein Oxid des Molybdäns bildet,

ist,
wobei unter einem Oxid des Molybdäns eine Substanz verstanden wird, die zu $\geq 98$ Gew.-% aus Mo und O besteht.

2. Verfahren nach Anspruch 1, **dadurch gekennzeichnet, dass** die feinteilige Substanz S $MoO_3$ ist.

3. Verfahren nach Anspruch 1 oder 2, **dadurch gekennzeichnet, dass** die spezifische Oberfläche $O_M$ der feinteiligen Substanz S $\geq 0,1$ m$^2$/g und $\leq 5$ m$^2$/g beträgt.

4. Verfahren nach einem der Ansprüche 1 bis 3, **dadurch gekennzeichnet, dass** der Teilchendurchmesser $d_{50}$ der feinteiligen Substanz S $\geq 1$ $\mu$m und $\leq 200$ $\mu$m beträgt.

5. Verfahren nach einem der Ansprüche 1 bis 4, **dadurch gekennzeichnet, dass** die aktive Masse in einer Schichtdicke von 100 bis 300 $\mu$m auf die Oberfläche des Trägerkörpers aufgebracht wird.

6. Verfahren nach einem der Ansprüche 1 bis 5, **dadurch gekennzeichnet, dass** die aktive Masse, bezogen auf die Gesamtmenge an Mo und V enthaltendem feinteiligem Multielementoxid, $\geq 1$ und $\leq 30$ Gew.-% an feinteiliger Substanz S enthält.

7. Verfahren nach einem der Ansprüche 1 bis 6, **dadurch gekennzeichnet, dass** das Bindemittel eine Lösung aus 20 bis 90 Gew.-% Wasser und 10 bis 80 Gew.-% einer organischen Verbindung ist.

8. Katalysatoren, die aus einem Trägerkörper und einer auf die Oberfläche des Trägerkörpers aufgebrachten katalytisch aktiven Masse sowie gegebenenfalls Bindemittel bestehen, wobei die katalytisch aktive Masse ein feinteiliges Gemisch aus

- wenigsten einem die Elemente Mo und V enthaltenden feinteiligen Multielementoxid
und
- wenigstens einer feinteiligen Substanz S ausgewählt aus der Gruppe bestehend aus Oxiden des Molybdäns und aus Verbindungen des Molybdäns, aus denen sich unter der Einwirkung von erhöhter Temperatur und molekularem Sauerstoff ein Oxid des Molybdäns bildet,

ist,
wobei unter einem Oxid des Molybdäns eine Substanz verstanden wird, die zu $\geq 98$ Gew.-% aus Mo und O besteht.

9. Verfahren der heterogen katalysierten partiellen Gasphasenoxidation von Acrolein zu Acrylsäure, **dadurch gekennzeichnet, dass** der Katalysator ein Katalysator gemäß Anspruch 8 ist.


**Claims**

1. A process for preparing a catalyst consisting of a support body and a catalytically active composition applied on the surface of the support body, in which the active composition is attached to the surface of the support body with the aid of a binder, wherein the active composition is a finely divided mixture of

- at least one finely divided multielement oxide comprising the elements Mo and V
and
- at least one finely divided substance S selected from the group consisting of oxides of molybdenum and of compounds of molybdenum from which an oxide of molybdenum is formed under the action of elevated temperature and molecular oxygen,

where an oxide of molybdenum is understood to mean a substance which consists of Mo and O to an extent of $\geq$ 98% by weight.

2.  The process according to claim 1, wherein the finely divided substance S is $MoO_3$.

3.  The process according to claim 1 or 2, wherein the specific surface area $O_M$ of the finely divided substance S is $\geq$ 0.1 $m^2/g$ and $\leq$ 5 $m^2/g$.

4.  The process according to any of claims 1 to 3, wherein the particle diameter $d_{50}$ of the finely divided substance S is $\geq$ 1 $\mu$m and $\leq$ 200 $\mu$m.

5.  The process according to any of claims 1 to 4, wherein the active composition is applied to the surface of the support body in a layer thickness of from 100 to 300 $\mu$m.

6.  The process according to any of claims 1 to 5, wherein the active composition, based on the total amount of finely divided multielement oxide comprising Mo and V, comprises $\geq$ 1 and $\leq$ 30% by weight of finely divided substance S.

7.  The process according to any of claims 1 to 6, wherein the binder is a solution composed of from 20 to 90% by weight of water and from 10 to 80% by weight of an organic compound.

8.  A catalyst which consists of a support body and a catalytically active composition applied to the surface of the support body, and also binders if appropriate, the catalytically active composition being a finely divided mixture of

    - at least one finely divided multielement oxide comprising the elements Mo and V
    and
    - at least one finely divided substance S selected from the group consisting of oxides of molybdenum and of compounds of molybdenum from which an oxide of molybdenum is formed under the action of elevated temperature and molecular oxygen,

    where an oxide of molybdenum is understood to mean a substance which consists of Mo and O to an extent of $\geq$ 98% by weight.

9.  A process for heterogeneously catalyzed partial gas phase oxidation of acrolein to acrylic acid, wherein the catalyst is a catalyst according to claim 8.

**Revendications**

1.  Procédé de fabrication d'un catalyseur constitué par un corps support et une masse catalytiquement active appliquée sur la surface du corps support, selon lequel la masse active est fixée à l'aide d'un liant sur la surface du corps support, **caractérisé en ce que** la masse active est un mélange finement divisé

    - d'au moins un oxyde de plusieurs éléments finement divisé contenant les éléments Mo et V,
    et
    - d'au moins une substance finement divisée S choisie dans le groupe constitué par les oxydes de molybdène et les composés de molybdène à partir desquels un oxyde de molybdène se forme sous l'effet d'une température élevée et d'oxygène moléculaire,

    un oxyde de molybdène se rapportant à une substance qui est constituée de $\geq$ 98 % en poids de Mo et O.

2.  Procédé selon la revendication 1, **caractérisé en ce que** la substance finement divisée S est $MoO_3$.

3.  Procédé selon la revendication 1 ou 2, **caractérisé en ce que** la surface spécifique $O_M$ de la substance finement divisée S est $\geq$ 0,1 $m^2/g$ et $\leq$ 5 $m^2/g$.

4.  Procédé selon l'une quelconque des revendications 1 à 3, **caractérisé en ce que** le diamètre de particule $d_{50}$ de la substance finement divisée S est $\geq$ 1 $\mu$m et $\leq$ 200 $\mu$m.

**5.** Procédé selon l'une quelconque des revendications 1 à 4, **caractérisé en ce que** la masse active est appliquée en une épaisseur de couche de 100 à 300 $\mu$m sur la surface du corps support.

**6.** Procédé selon l'une quelconque des revendications 1 à 5, **caractérisé en ce que** la masse active contient, par rapport à la quantité totale d'oxyde de plusieurs éléments finement divisé contenant Mo et V, $\geq 1$ et $\leq 30$ % en poids de substance finement divisée S.

**7.** Procédé selon l'une quelconque des revendications 1 à 6, **caractérisé en ce que** le liant est une solution de 20 à 90 % en poids d'eau et 10 à 80 % en poids d'un composé organique.

**8.** Catalyseurs, qui sont constitués par un corps support et une masse catalytiquement active appliquée sur la surface du corps support, ainsi qu'éventuellement un liant, la masse catalytiquement active étant un mélange finement divisé

- d'au moins un oxyde de plusieurs éléments finement divisé contenant les éléments Mo et V,
et
- d'au moins une substance finement divisée S choisie dans le groupe constitué par les oxydes de molybdène et les composés de molybdène à partir desquels un oxyde de molybdène se forme sous l'effet d'une température élevée et d'oxygène moléculaire,

un oxyde de molybdène se rapportant à une substance qui est constituée de $\geq 98$ % en poids de Mo et O.

**9.** Procédé d'oxydation partielle en phase gazeuse sous catalyse hétérogène d'acroléine en acide acrylique, **caractérisé en ce que** le catalyseur est un catalyseur selon la revendication 8.

Figur 1

EP 2 134 465 B1

Figur 2

Figur 3

## IN DER BESCHREIBUNG AUFGEFÜHRTE DOKUMENTE

### In der Beschreibung aufgeführte Patentdokumente

- WO 9511081 A
- WO 2004108267 A
- WO 2004108284 A
- US 20060205978 A
- EP 714700 A
- DE 102005010645 A
- DE 10350822 A
- DE 102004025445 A
- EP 990636 A
- EP 1106598 A
- DE 10232748 A
- EP 614872 A
- WO 0224327 A
- EP 72448 A
- DE 1642921 A
- DE 2106796 A
- DE 2626887 A
- DE 2909670 A
- DE 2526238 A
- US 3956377 A
- DE 235151 A
- DE 2909671 A
- DE 19835247 A
- DE 10122027 A
- DE 10051419 A
- DE 10046672 A
- DE 10119933 A
- DE 10307983 A
- DE 19948523 A
- DE 19910508 A
- EP 700714 A
- EP 700893 A
- DE 19910506 A
- DE 19948241 A
- DE 2830765 C
- DE 2513405 C
- US 3147084 A
- DE 2201528 A
- EP 383224 A
- DE 2903218 A
- EP 468290 B
- US 20060161019 A

### In der Beschreibung aufgeführte Nicht-Patentliteratur

- Synthese und strukturelle Untersuchungen von Molybdän-, Vanadium- und Wolframoxiden als Referenzverbindungen für die heterogene Katalyse. **DR. ANDREAS BLUME.** Dissertation. Fakultät II Mathematik- und Naturwissenschaften der Technischen Universität, 2004
- *Surface Science,* 1993, vol. 292, 261-6
- *J. Solid State Chem.,* 1996, vol. 124, 104